Europäisches Patentamt

(19) European Patent Office

Office européen des brevets

(11) Veröffentlichungsnummer : **0 250 498 B1**

(12) **EUROPÄISCHE PATENTSCHRIFT**

(45) Veröffentlichungstag der Patentschrift :
03.07.91 Patentblatt 91/27

(51) Int. Cl.⁵ : **A61H 31/00,** A61F 2/02,
A61K 35/60

(21) Anmeldenummer : 87900060.2

(22) Anmeldetag : 18.12.86

(86) Internationale Anmeldenummer :
PCT/DE86/00518

(87) Internationale Veröffentlichungsnummer :
WO 87/03802 02.07.87 Gazette 87/14

(54) **VORRICHTUNG FÜR DIE AUFNAHME UND ZUM EINBRINGEN VON GEWEBEN IN DEN MENSCHLICHEN KÖRPER SOWIE HIERFÜR VERWENDBARES INSTRUMENT.**

(30) Priorität : 20.12.85 DE 3545237

(43) Veröffentlichungstag der Anmeldung :
07.01.88 Patentblatt 88/01

(45) Bekanntmachung des Hinweises auf die
Patenterteilung :
03.07.91 Patentblatt 91/27

(84) Benannte Vertragsstaaten :
AT CH DE FR GB IT LI NL SE

(56) Entgegenhaltungen :
EP-A- 0 046 669
FR-A- 2 384 504
GB-A- 2 046 209
US-A- 3 093 831
US-A- 3 804 088

(56) Entgegenhaltungen :
US-A- 3 946 734
US-A- 4 479 796
Chemical Abstracts, Band 101, 1984 (Columbus, Ohio, USA), P. Ronner et al., "Difference in glucose dependency of insulin and somatostatin release", siehe Seite 106, Zusammenfassung Nr. 66483v, Am. J. Physiol.1984, 246(6, Pt.1), E506-E509

(73) Patentinhaber : SCHREZENMEIR, Jürgen
Backhaushohl 24
W-6500 Mainz (DE)

(72) Erfinder : SCHREZENMEIR, Jürgen
Backhaushohl 24
W-6500 Mainz (DE)

(74) Vertreter : Pöhner, Wilfried Anton, Dr.
Kaiserstrasse 27 Postfach 63 23
W-8700 Würzburg 1 (DE)

## Beschreibung

Die Erfindung betrifft eine Vorrichtung zum Einbringen von Geweben in den menschlichen Körper, wobei das Gewebe Insulin bildet und sich im Innerem eines geschlossenen Schlauches befindet, der zumindest teilweise aus einer Membran besteht, durch die die Körperflüssigkeiten hindurchdiffundieren, Antikörper und Abwehrzellen jedoch zurückgehalten werden sowie ein Instrument zur Einbringung derselben.

Aus der US-A-3,093,831 sind Vorrichtungen gattungsgemäßer Art bekannt, in deren Inneren Gewebe von Drüsen angeordnet ist, welches mit Hilfe der Vorrichtung in den lebenden menschlichen Organismus implantiert wird. Zur Unterbindung der dabei auftretenden Immunreaktionen, die auf ein Abstoßen des als körperfremd erkannten Gewebes hinarbeiten, werden Vorrichtungen mit semipermeabler Membran vorgeschlagen, deren Porenweite so gewählt ist, daß Antigene und Antikörper nicht hindurchtreten können. Jedoch werden die Nährstoffe auf der das Implantat umgebenden Blutflüssigkeit hindurchgelassen und können so zum Gewebe gelangen. Derartige Vorrichtungen können insbesondere auch insulinerzeugendes Gewebe der Bauspeicheldrüse aufnehmen, welches zur Implantation bei zuckerkranken Patienten bestimmt ist. Das nutzbare Zellgewebe der Langehansinseln ist über die gesamte Bauchspeicheldrüse verstreut, so daß es außerordentlich mühselig und kostenaufwendig ist, diese zu isolieren und einzusammeln.

Hiervon ausgehend hat sich die Erfindung die Schaffung einer möglichst preisgünstig und einfach beschaffbaren Vorrichtung zur Aufgabe gemacht, die sich zur Implantation bei zuckerkranken Patienten eignet.

Gelöst wird diese Aufgabe dadurch, daß Gewebe von der Blutversorgung unabhängig ist und aus Zellgewebe von Teleostien (Knochenfischen) gewonnenen Brockmann-Bodies besteht.

Der entscheidende Vorteil ist darin zu sehen, daß bei Knochenfischen das insulinerzeugende Zellgewebe zusammenhängend und nicht mehr verstreut auftritt und daß durch dieses Gewebe erzeugte Insulinhormon problemlos für Menschen einsetz- und nutzbar ist. Es resultiert eine wesentliche Vereinfachung der Isolierung und Gewinnung und damit der Herstellung. Die Durchlässigkeit und "Maschenweite" der Membran ist so zu wählen, daß Körperflüssigkeiten hindurchdiffundieren, Antikörper und Abwehrzellen jedoch zurückgehalten werden. In Anlehnung an die übliche Terminologie sind hierbei unter "Antikörper und Abwehrzellen" all jene Zellen gemeint, die Abstoßungsreaktionen auslösen und Elimination des körperfremden Gewebes zu bewirken versuchen. Unter den Sammelbegriff "Körperflüssigkeit" fallen die Nährstoffe, Hormone, insbesondere auch Regulationshormone und alle anderen Stoffe, die zur Aufrechterhaltung des Lebens in den Gewebezellen erforderlich sind.

Grundsätzlich ist im Sinne der Erfindung die Größe des in der erfindungsgemäßen Vorrichtung befindlichen Gewebes weit auszulegen, denn es sollen sowohl Gewebeeinzelzellen als auch größere Gewebeteile mit einer Vielzahl an Zellen umfaßt werden. Des weiteren ist die Form des Schlauches beliebig und sie kann als Beutel oder Kammer oder gar als Kapsel ausgeformt sein. In funktioneller Hinsicht geht es im wesentlichen darum, das Gewebe mit einer geschlossenen Hülle von entsprechender Durchlässigkeit zu umgeben.

Die Benutzung geschieht in der Weise, daß die erfindungsgemäße Vorrichtung Beispielsweise durch einen kleinen operativen Eingriff in den Körper eingebracht wird und dort verbleibt und durch Diffusion der Körperflüssigkeit durch die Membran ernährt und am Leben gehalten wird. Dabei erzeugt das Gewebe Hormone, die durch die Membran jedoch in zur Körperflüssigkeit entgegengesetzter Richtung hindurchdiffundieren. Aus der Tatsache, daß Körperflüssigkeit umter anderem bereits Regulationshormone enthält, ergibt sich zwingend, daß auch die durch das Gewebe erzeugten Hormone die Membran durchdringen können und in den Körper des Patienten gelangen. Gleichzeitig verhindert die Membran, daß die sich in Patienten auf Grund des eingebrachten, körperfremden Gewebes ausbildenden Abwehrzellen und Antikörper zurückgehalten werden, so daß die Funktionsfähigkeit. des implantierten Gewebes nicht beeinträchtigt wird.

Bei Zuckerkranken besonders wichtig ist, daß durch Implantierung eines insulinerzeugenden Gewebes dem Körper des Patienten kontinuierlich Hormone zugeführt werden, so daß sich der Wert des Blutzuckerspiegels permanent im Normalbereich bewegt.
Ein Zuführen und Spritzen von Insulin erübrigt sich dann und der Patient kann ohne ständige Überwachung seines Blutzuckerspiegels leben.

Im implantierten Zustand wird sich die Oberfläche des Schlauches und insbesondere die der Membran nach gewisser Zeit mit Gewebe zusetzen und zuwachsen. Da hierdurch die Diffusion wesentlich erschwert oder völlig unterbunden wird, muß die erfindungsgemäße Vorrichtung von Zeit zu Zeit ausgetauscht werden. Aus diesem Grund schlägt die Erfindung in einer Weiterbildung vor, den Schlauch von zylindrischer Gestalt zu wählen und in etwa zentral einen Faden anzuordnen, dessen eines Ende an einer als Kappe oder Schild ausgebildeten Stirnseite befestigt und dessen gegenüberliegendes Ende die andere Stirnseite durchgreift und in einem gewissen Abstand davor endet. Der etwa zentral verlaufende Faden läßt eine problemlose Austausch- und Auswechselbarkeit der gesamten Vorrichtung dadurch zu, daß er an seinem freien Ende ergriffen und durch Zug die gesamte Vorrichtung aus dem Körper des Patienten entfernbar ist. Da das andere Ende des Fadens an der hinteren, gegenüberliegenden und als Kappe oder Schild ausgebildeten Stirnseite befestigt ist und damit dieser Vorrichtungsteil eine entsprechende Verstärkung erfährt, lassen sich die zum Auswechseln erforderlichen Kräfte auf die gesamte Vorrichtung übertragen. Durch die Tatsache, daß der Faden durch die gegenüberliegende Stirnseite verläuft und in einem gewissen Abstand davor endet, läßt er sich leicht erfassen und die gesamte Vorrichtung durch Zug bewegen und entfernen.

In einer zweckmäßigen Weiterbildung ist hierzu am freien Ende des Fadens ein Knopf oder eine Öse angebracht, welche das Ergreifen des Fadenendes wesentlich erleichtert und vereinfacht. Auch diese Maßnahme stellt einen Beitrag zur Verbesserung der Auswechsel- und Austauschbarkeit der erfindungsgemäßen Vorrichtung dar.

Der Faden ist so zu wählen, daß er zur Aufnahme hoher Zugbelastungen in der Lage ist und zum anderen auch bei längerer Verweildauer im Körper des Patienten keine unerwünschten oder schädlichen Reaktionen auftreten können. Aus diesem Grunde empfiehlt die Erfindung das Material Kunststoff oder Metall.

Im Hinblick auf die Ausbildung des Schlauches wird durch die Erfindung in einer Ausführungsform die Verwendung einer Hohlfaser, wie z.B. eines Dialyse-Schlauches, vorgeschlagen und bezüglich der Ausbildung der Membran die Verwendung einer Dialyse-Membran empfohlen. Sowohl Dialyse-Schlauch als auch Dialyse-Membran erfüllen die im Hinblick auf die Durchlässigkeit durch die Erfindung gestellten Anforderungen.

Grundsätzlich besteht die Möglichkeit, daß der das Gewebe umgebende Schlauch in seiner Gesamtheit die Membran darstellt. Für derartige Fälle schlägt die Erfindung die Erzeugung der Membran durch das an sich bekannte Verfahren der Enkapsulierung vor, bei dem die Herstellung in drei Verfahrensschritten erfolgt : Beim ersten wird das Gewebe von einem Gel umgeben, im zweiten Arbeitsschritt wird auf diesem Gel ein Netzwerk aus Aminosäuren oder anderen Polymeren hergestellt, so daß man im Ergebnis das Gewebe umgeben von einer Membran erhält, welche auf chemischen Wege durch Vernetzung von Polymeren, wie z.B. Aminosäuren odgl. erzeugt wurde. Ein wesentlicher Vorteil dieser bevorzugten Ausführungsform besteht darin, daß es nunmehr möglich ist kleinste Gewebeteile bis hin zu einzelnen Zellen mit einer Membran zu umgeben, wodurch sich kleinste Abmessungen erreichen lassen, die das Einspritzen in den Körper des Patienten erlauben, so daß sich der operative Eingriff zur Implantierung erübrigt.

Eine wesentliche Vereinfachung der Anbringung der erfindungsgemäßen Vorrichtung oder auch anderer Implantate, wie z.B. Depotmedikamente, Strahlenquellen usw. in dem Körper des Patienten läßt sich durch ein ebenfalls durch die Erfindung vorgeschlagenes Instrument erreichen, welches aus einer Nadel, insbes. aus Metall, besteht, an die sich eine zylindrische Hülle endseitig anschließt, in dessen Inneren der Schlauch mit dem Gewebe, also die erfindungsgemäße Vorrichtung, anordenbar ist. Sie ermöglicht ohne aufwendige Operation die Implantierung der erfindungemäßen Vorrichtung dadurch, daß sie in den Körper, beispielsweise in die Bauchdecke, eingeführt wird und mit Erreichen der gewünschten Position der innerhalb der Hülle befindlichen Vorrichtung diese dort fixiert wird, hingegen die Metallnadel weitergeführt wird, die vorzugsweise an einer anderen Stelle, dann jedoch mit leerer Hülle den Körper verläßt.

Besonders einfach wird das Fixieren innerhalb des Körpers, wenn der Faden in Bezug auf die Hülle so gewählt ist, daß er endseitig übersteht, weil dann für den Benutzer die Möglichkeit gegeben ist, einzig und allein durch Festhalten des Fadens die erfindungsgemäße Vorrichtung auch bei Fortsetzung der Bewegung der Nadel auf einfache Weise zu fixieren.

Durch die schließlich noch vorgeschlagene Verwendung einer gekrümmten Nadel erreicht man eine Erleichterung der Handhabung, dahingehend, daß die gekrümmte Nadel bereits in geringer Entfernung von der Einstichstelle wieder den Körper verlassen kann.

Weitere Einzelheiten, Merkmale und Vorteile der Erfindung lassen sich dem nachfolgenden Beschreibungsteil entnehmen, in dem die in schematischer Darstellung in der Zeichnung wiedergegebenen Ausführungsbeispiele näher erläutert sind. Es zeigen :

Figur 1   eine erfindungsgemäße Vorrichtung für die Aufnahme und zum Einbringen von Gewebe in den menschlichen Körper in prinzipienhafter Querschnittsdarstellung,

Figur 2   ein zum Einbringen verwendbares Instrument ebenfalls in prinzipienhafter Darstellung.

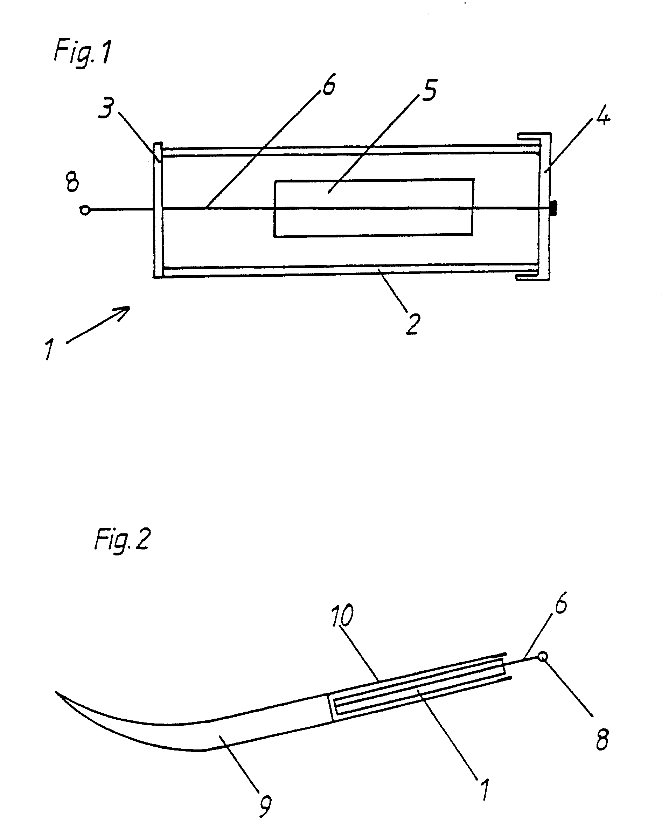

Der in Figur 1 gezeigte Schlauch 1 wird in seiner Gesamtheit gebildet durch einen Zylinder 2, der an seinem linken Ende durch eine Stirnseite 3 und an seinem rechten über eine Kappe 4 verschlossen ist, so daß man im Ergebnis ein nach allen Seiten geschlossenes Gebilde erhält. In dessen Inneren befindet sich ein hier nur andeutungsweise wiedergegebenes Gewebe 5 sowie ein Faden 6, der im wesentlichen zentral verläuft, und mit seinem einen Ende an der Kappe 4 befestigt ist, gleichzeitig die linke Stirnseite 3 einer hier nicht eingezeichneten abgedichteten Öffnung durchgreift und vor derselben in einem gewissen Abstand in einer Öse 8 endet. Dieses freie Überstehen des Fadens 6 sowie die Anbringung der Öse 8 erleichtern das Ergreifen des Fadens 6, mit dessen Hilfe sich auf Zugbeanspruchung die gesamte Vorrichtung translatorisch in Fadenrichtung bewegen läßt. Ein leichtes Entfernen und damit Austauschen der bereits implantierten Vorrichtung läßt sich mit dessen Hilfe durchführen.

In Figur 2 ist ein Instrument dargestellt, mit dessen Hilfe die beispielsweise in Figur 1 beschriebene Vorrichtung in den Körper des Patienten eingebracht werden kann. Diese besteht aus einer gekrümmten Metallnadel 9, an deren äußeren Ende sich eine zylindrische Hülle 10 anschließt. In dieser befindet sich der Schlauch 1, der der Hülle 10 so zugeordnet ist, daß das überstehende Ende des Fadens 6 und insbesondere die daran befindliche Öse 8 ebenfalls über die Hülle 10 hinausragen. Dies erleichtert das Erfassen des Fadens 6 und insbesondere der Öse 8, wodurch dann der Schlauch 1 in der momentan befindlichen Postition fixiert werden kann, insbesondere auch dann, wenn die Metallnadel 9 weiterbewegt wird.

Die Handhabung dieses Instrumentes ist wie folgt : Als erstes wird in die Hülle 10 die erfindungsgemäße Vorrichtung und insbesondere der Schlauch 1 eingeführt, dann wird die Nadel 9 in die zur Aufnahme des Implantats bestimmte Körperpartie eingestochen und zwar soweit, bis der Schlauch 1 in der gewünschten Endposition zu liegen kommt. Dort erfolgt seine Fixierung dadurch, daß der faden 6 vermittels der Öse 8 festgehalten wird und demzufolge auch der Schlauch 1 bei Weiterbewegung der Nadel 9 raumfest bleibt. Durch deren Krümmung wird erreicht, daß die Nadel bereits in geringer Entfernung von der Einstichstelle wieder den Körper verläßt. Ein aufwendiger, operativer Eingriff zur Implantation ist dann überflüssig.

Im Ergebnis gibt die Erfindung eine Vorrichtung an die Hand, mit deren Hilfe auf unproblematische Weise Gewebe implantiert werden kann, ohne daß es der Körper abstößt.

## Ansprüche

1. Vorrichtung zum Einbringen von Geweben in den menschlichen Körper, wobei das Gewebe Insulin bildet und sich im Inneren eines geschlossenen Schlauches befindet, der zumindest teilweise aus einer Membran besteht, durch die die Körperflüssigkeiten hindurchdiffundieren, Antikörper und Abwehrzellen jedoch zurückgehalten werden, **dadurch gekennzeichnet,** daß das Gewebe von der Blutversorgung unabhängig ist und aus Zellgewebe von Teleostien (Knochenfische) gewonnene Brockmann-Bodies sind.

2. Vorrichtung nach Anspruch 1, **gekennzeichnet durch** einen Schlauch 1 von zylindrischer Gestalt, in dem etwa zentral ein Faden 6 verläuft, dessen eines Ende an eine als Kappe 4 oder Schild ausgebildeten Stirnseite befestigt und dessen gegenüberliegendes Ende die andere Stirnseite 3 durchgreift und in gewissem Abstand davor endet.

3. Vorrichtung nach Anspruch 2, **dadurch gekennzeichnet,** daß am freien Ende des Fadens 6 ein Knopf oder eine Öse 8 angebracht ist.

4. Vorrichtung nach Anspruch 2 oder 3, **gekennzeichnet durch** einen Faden 6 aus Kunststoff oder Metall.

5. Vorrichtung nach einem der Ansprüche 1 bis 4, **dadurch gekennzeichnet,** daß der Schlauch 1 eine Hohlfaser, z.B. ein Dialyse-Schlauch, und/oder die Membran eine Dialyse-Membran ist.

6. Vorrichtung nach Anspruch 1, **gekennzeichnet durch** Enkapsulierung des Gewebes 5.

7. Instrument zur Einbringung der Vorrichtung gemäß einem der Ansprüche 1 bis 6 in den Körper des Patienten zusammen mit der Vorrichtung, **dadurch gekennzeichnet,** daß sich an eine Nadel 9, insbesondere Metallnadel, eine zylindrische Hülle 10 endseitig anschließt, in deren Inneren der Schlauch 1 mit dem Gewebe 5 angeordnet ist.

8. Instrument nach Anspruch 7, **dadurch gekennzeichnet,** daß der Faden 6 über die Hülle 10 hinausragt.

9. Instrument nach Anspruch 7 oder 8, **gekennzeichnet durch** eine gekrümmte Nadel 9.

## Claims

1. Device for the insertion of tissue into the human body, whereby the tissue produces insulin and is disposed inside a closed tube, which comprises at least in part a membrane through which body fluids can diffuse, but which holds back antibodies and defence cells **wherein** said tissue is independent of the blood supply and is made of Brockmann Bodies won from cell tissue from teleostei (bony fish).

2. Device according to claim 1 **wherein** there is a cylindrically shaped tube 1, through which passes a thread 6 approximately in the centre, one end of which is fixed to an end wall shaped as a cap 4 or shield and whose opposite end penetrates the other end wall 3 to end at a certain distance beyond.

3. Device according to claim 2 **wherein** a head or eyelet 8 is disposed on the free end of the thread 6.

4. Device according to claim to 2 or 3 **wherein** said thread 6 is made of metall or plastic.

5. Device according to one of claims 1 to 4 **wherein** said tube 1 is a hollow fibre, for example, a dialysis tube, and/or the membrane is a dialysis membrane.

6. Device according to claim 1 **wherein** said tissue 5 is encapsulated.

7. Instrument for the insertion of the device according to one of claims 1 to 6 into the body of the patient together with the device **wherein** the end side of said cylindrical cover 10 is connected to a needle 9, especially a metal needle, inside the cover being disposed said tube 1 with said tissue 5.

8. Instrument according to claim 7 **wherein** said thread 6 projects beyond said cover 10.

9. Instrument according to claim 7 or 8 **wherein** the needle 9 is curved.

## Revendications

1. Dispositif destiné à introduire des tissus dans le corps humain, lesdits tissus produisant de l'insuline et se trouvant à l'intérieur d'un tuyau flexible fermé, constitué du moins en partie d'une membrane à travers laquelle diffusent les liquides du corps, alors que les anticorps et les cellules défensives sont retenus, caractérisé en ce que le tissu est indépendant de l'alimentation en sang et qu'il est constitué de tissu cellulaire de Brockmann-Bodies obtenus à partir de téléostéens (poissons osseux).

2. Dispositif selon la revendication 1, caractérisé par le tuyau flexible 1 de configuration cylindrique, dans lequel est disposé dans une position plus ou moins centrale un fil 6 dont une extrémité est fixée à un côté frontal conçu en forme de capuchon 4 ou de bouclier et dont l'extrémité opposée passe à travers l'autre côté frontal 3 et se termine à un certain écart devant ce front.

3. Dispositif selon la revendication 2, caractérisé en ce que l'extrémité libre du fil 6 est munie d'un bouton ou d'un oeillet 8.

4. Dispositif selon la revendication 2 ou 3, caractérisé par un fil 6 en matière synthétique ou métal.

5. Dispositif selon l'une quelconque des revendications de 1 à 4, caractérisé en ce que le tuyau flexible 1 est une fibre creuse, p.ex. un tuyau flexible à dialyse, et/ou que la membrane est une membrane à dialyse.

6. Dispositif selon la revendication 1, caractérisé par l'encapsulement du tissu 5.

7. Instrument destiné à introduire le dispositif selon l'une quelconque des revendications de 1 à 6 avec le dispositif même dans le corps du malade, caractérisé en ce qu'une gaine 10 est rattachée à l'extrémité d'une aiguille 9, notamment une aiguille métallique, et que le tuyau flexible 1 avec le tissu 5 est disposé à l'intérieur de cette gaine.

8. Instrument selon la revendication 7, caractérisé en ce que le fil 6 dépasse la gaine 10.

9. Instrument selon la revendication 7 ou 8, caractérisé par une aiguille courbée 9.

Fig.1

Fig.2